Europäisches Patentamt

European Patent Office

Office européen des brevets

(11) Veröffentlichungsnummer: **0 170 779**
**B1**

## (12) EUROPÄISCHE PATENTSCHRIFT

(45) Veröffentlichungstag der Patentschrift:
**22.06.88**

(21) Anmeldenummer: **85104108.7**

(22) Anmeldetag: **04.04.85**

(51) Int. Cl.⁴: **A 61 F 2/30,** A 61 F 2/38,
F 16 B 13/04

(54) Dübelartige Verankerung für eine zementfreie Fixierung von Knochenimplantaten.

(30) Priorität: **02.07.84 CH 3170/84**

(43) Veröffentlichungstag der Anmeldung:
**12.02.86 Patentblatt 86/7**

(45) Bekanntmachung des Hinweises auf die Patenterteilung:
**22.06.88 Patentblatt 88/25**

(84) Benannte Vertragsstaaten:
**AT DE FR GB IT**

(56) Entgegenhaltungen:
**EP - A - 0 077 868**
**DE - A - 1 902 066**
**DE - A - 1 954 414**
**FR - A - 1 259 138**
**FR - A - 2 278 008**
**US - A - 2 381 050**

(73) Patentinhaber: **GEBRÜDER SULZER
AKTIENGESELLSCHAFT, Zürcherstrasse 9,
CH-8401 Winterthur (CH)**

(72) Erfinder: **Karpf, Kurt, Alte Strasse 175,
CH-4718 Holderbank (CH)**

(74) Vertreter: **Dipl.-Ing. H. Marsch Dipl.-Ing. K. Sparing
Dipl.-Phys.Dr. W.H. Röhl Patentanwälte,
Rethelstrasse 123, D-4000 Düsseldorf (DE)**

## Beschreibung

Die Erfindung betrifft eine dübelartige Verankerung für eine zementfreie Fixierung von Knochenimplantaten, insbesondere von Femur und/oder Tibiateilen von künstlichen Kniegelenken, bestehend aus einem einseitig offenen, mit Hilfe von Längsschlitzen aufspreizbaren Hohlkörper und einem in den Hohlkörper eintreibbaren Spreizkörper, wobei mindestens der Hohlkörper auf seiner Aussenmantelfläche widerhakenartige Verzahnungen trägt.

Verankerungen der vorstehend genannten Art sind beispielsweise aus der CH-A-566 770 bekannt. Sie bilden dort die Verankerung eines Köchers, der zur Aufnahme des Schaftes eines Kleingelenkes, beispielsweise eines Fingergelenkes, dient. Der hohlzylindrische Köcher ist dabei an seinem freien Ende mit Längsschlitzen versehen; ein in den Hohlraum des Köchers im Bereich der Schlitze eingepresster Spreizkörper spreizt den Hohlkörper in diesem Bereich auf und presst die verbleibenden Mantelstege gegen die Wand einer operativ vorbereiteten Ausnehmung im Knochen.

Bei Kleingelenken, über die keine grossen Belastungen übertragen werden, reicht diese Art der zementfreien Verankerung aus. Für grosse Gelenke, beispielsweise Kniegelenke, bietet sie jedoch keine Gewähr für eine sichere Fixierung des Implantats. Aufgabe der Erfindung ist es daher, eine dübelartige Verankerung für eine zementfreie Fixierung zu schaffen, die auch für Implantate geeignet ist, die relativ grossen Belastungen gewachsen sein müssen.

Diese Aufgabe wird mit der vorliegenden Erfindung dadurch gelöst, dass der Spreizkörper in Form, Abmessungen und Lage an die zwischen den Längsschlitzen verbliebenen Mantelstege des Hohlkörpers angepasste Längsausnehmungen hat, zwischen denen ebenfalls mit einer widerhakenartigen Verzahnung versehene Vorsprünge hervorstehen, deren Aussendurchmesser an denjenigen des Hohlkörpers angepasst ist.

Mit der erfindungsgemässen Konstruktion wird für das anwachsende Gewebe auf dem ganzen Umfang der Verankerung – und ebenso über ihre ganze Höhe – ein praktisch geschlossener «Schaft» dargeboten, bei dem die Primär-Verankerung durch die aufgespreizten Mantelstege des Hohlkörpers gewährleistet ist. Bei der Implantation kann dabei sowohl der Hohlkörper auf den bereits in einer Knochenbohrung gelagerten Spreizkörper aufgeschoben als auch umgekehrt der Spreizkörper in den in eine Bohrung eingesetzten Hohlkörper eingetrieben werden.

Das für eine sichere Primär-Verankerung notwendige Aufspreizen des Hohlkörpers, dessen Längsschlitze möglichst weitgehend sich über seine ganze Höhe erstrecken, lässt sich bei der Implantation erreichen, wenn sich der Kern des Spreizkörpers gegen sein Kopfende hin stetig erweitert oder wenn – ähnlich dem bekannten Körper – das freie Ende des Hohlkörpers an seinem Innendurchmesser konisch verjüngt ist. Auf diese Weise kann der Hohlkörper ohne Beschädigung des Knochens in relativ eng an seinen Durchmesser angepasste Bohrungen eingeführt werden. Er weitet sich dann erst auf, wenn er praktisch vollständig auf den Spreizkörper aufgeschoben bzw. dieser praktisch vollständig in ihn eingeschoben ist.

Die gegenseitige Haftung zwischen Hohlkörper und Spreizkörper lässt sich verbessern, wenn die Innenfläche der Mantelstege des Hohlkörpers eine widerhakenartige Mikroverzahnung trägt. Weiterhin kann es sich als vorteilhaft erweisen, wenn der Hohlkörper aus Metall, insbesondere aus Titan oder einer Titanlegierung, und der Spreizkörper aus Kunststoff, insbesondere aus Polyäthylen der Klassifikationen HDPE oder UHMW, bestehen. Selbstverständlich können jedoch für Spreizkörper auch andere in der Implantattechnik gebräuchliche Werkstoffe, wie z. B. Metalle, pyrolytischer Kohlenstoff, Keramik oder faserverstärkte Kunststoffe verwendet werden. Ebenso ist es möglich, den Hohlkörper aus allen Implantatwerkstoffen herzustellen, sofern sie die notwendige Festigkeit und Elastizität haben.

Im folgenden wird die Erfindung anhand von Ausführungsbeispielen im Zusammenhang mit der Zeichnung näher erläutert.

Fig. 1 zeigt, teilweise im Schnitt und teilweise in einer Ansicht, eine erste Ausführungsform eines Spreizkörpers;

Fig. 2 ist der Schnitt II–II von Fig. 1;

Fig. 3 gibt einen zu dem Spreizkörper nach Fig. 1 passenden Hohlkörper, ebenfalls teilweise im Schnitt, wieder;

Fig. 4 ist ein Schnitt IV–IV von Fig. 3;

Fig. 5a und 5b stellen in ähnlicher Form wie Fig. 1 und 3 schematisch in einem Knochen ein Zwischenstadium bzw. das Endstadium bei der bzw. nach der Implantation einer Verankerung nach den Fig. 1 und 3 dar;

Fig. 6 und 7 sind die Schnitte VI–VI bzw. VII–VII von Fig. 5a bzw. Fig. 5b;

Fig. 8 gibt vergrössert ein Detail aus Fig. 5a wieder;

Fig. 9 ist in gleicher Darstellung wie Fig. 1 ein weiterer Spreizkörper;

Fig. 10 gibt einen zum Spreizkörper nach Fig. 9 gehörenden Hohlkörper wieder;

Fig. 11a und 11b stellen, wie Fig. 5a und 5b, Zwischen- und Endstadium der implantierten Verankerung nach Fig. 9 und 10 dar;

Fig. 12 schliesslich ist eine Ansicht von Fig. 10 von unten.

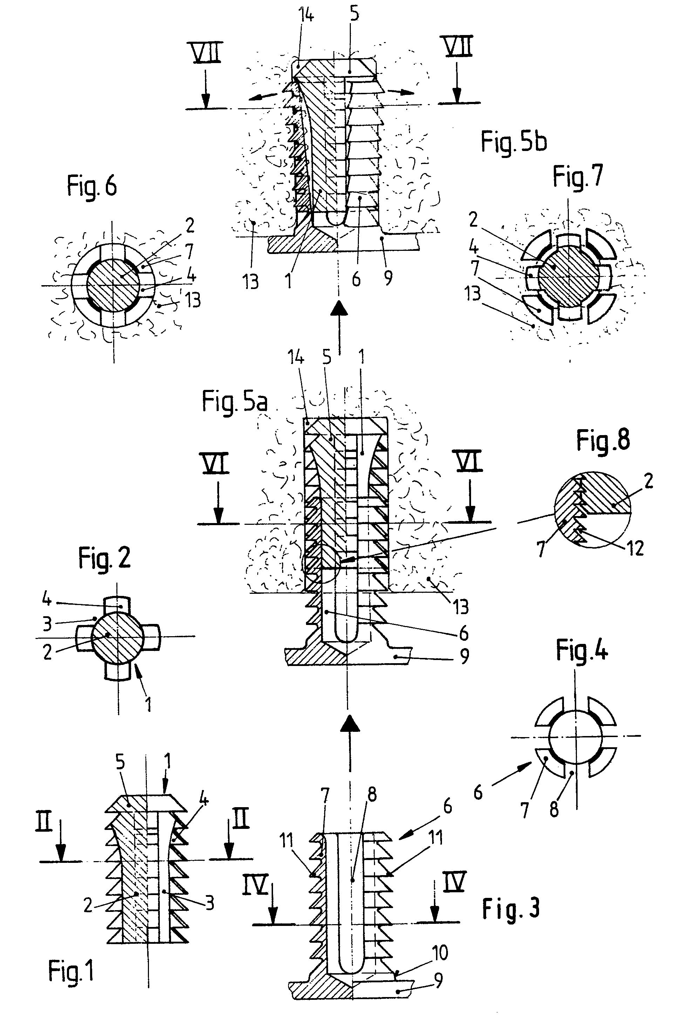

Der Spreizkörper 1 nach Fig. 1 und 2 hat einen massiven Kern 2, aus dem Vorsprünge 4 hervorstehen, die in Längsrichtung sägezahnartige Verzahnungen bilden. Zwischen den Vorsprüngen 4 sind Längsausnehmungen 3 vorhanden, die auf einer Seite offen sind und auf der anderen an einem plattenartigen Kopf 5 enden. Der Durchmesser des Kerns 2 erweitert sich, wie Fig. 1 wiedergibt, zum Kopf 5 hin im letzten Drittel der Kernlänge stetig.

Der zugehörige Hohlkörper 6 nach Fig. 3 und 4 wird gebildet von vier Mantelstegen 7, die durch Schlitze 8 getrennt und über einen Boden 10 an dem zu verankernden Implantat 9 gehalten sind. Die Mantelstege 7 sind aussen ebenfalls mit einer Sägezahn-Verzahnung 11 versehen.

Die Vorsprünge 4 und die Längsausnehmungen 3 des Spreizkörpers 1 und die Schlitze 8 und die Mantelstege 7 des Hohlkörpers 6 sind in Form und Abmessungen so aufeinander abgestimmt, dass die Vorsprünge 4 in die Schlitze 8 und die Mantelstege 7 in die Längsausnehmungen 3 passen. Nach dem gegenläufigen Ineinanderschieben bilden die Körper 1 und 6 auf diese Weise einen geschlossenen «Zapfen» (Fig. 6), an und in den allseitig Gewebe an- bzw. einwachsen kann.

Wie Fig. 8 zeigt, weist die Innenwand der Mantelstege 7 eine wiederum sägezahnartige Mikroverzahnung 12 auf, mit der sie sich im Kern 2 des Spreizkörpers 1 verhaken können.

Die «Montage» der neuen Verankerung, die beispielsweise für die Fixierung einer Schalenprothese für ein Kniegelenk in den Kondylen des Femurs eingesetzt wird, geschieht auf folgende Weise:

In den Knochen 13 wird zunächst eine Bohrung 14 eingebracht, deren Durchmesser dem Durchmesser des Kopfes 5 und der Vorsprünge 4 des Spreizkörpers 1 entspricht. Die Tiefe der Bohrung 14 ist so auf die Verankerung 1, 6 abgestimmt, dass das Implantat 9 nach der Implantation im Knochen 13 aufliegt, wenn die Mantelstege 7 bis nahe an den Kopf 5 auf den Spreizkörper 1 aufgeschoben sind (Fig. 5b).

Nach dem Einsetzen des Spreizkörpers 1 in die Bohrung 14 wird der Hohlkörper 6 mit seinen Mantelstegen 7 in die Längsausnehmungen 3 «eingefädelt» und, wie durch Pfeile angedeutet, auf den Spreizkörper 1 aufgeschoben bzw. aufgeschlagen (Fig. 5a). Gelangen die Mantelstege 7 dabei auf dem Spreizkörper 1 in den Längenbereich, in dem sich der Kerndurchmesser erweitert, so werden sie radial nach aussen gedrückt (Fig. 7) und dringen mit ihrer Verzahnung 11 in die Wand der Knochenbohrung 14 ein. Dadurch wird eine gute Primär-Fixierung des Implantates 9 sichergestellt (Fig. 5b).

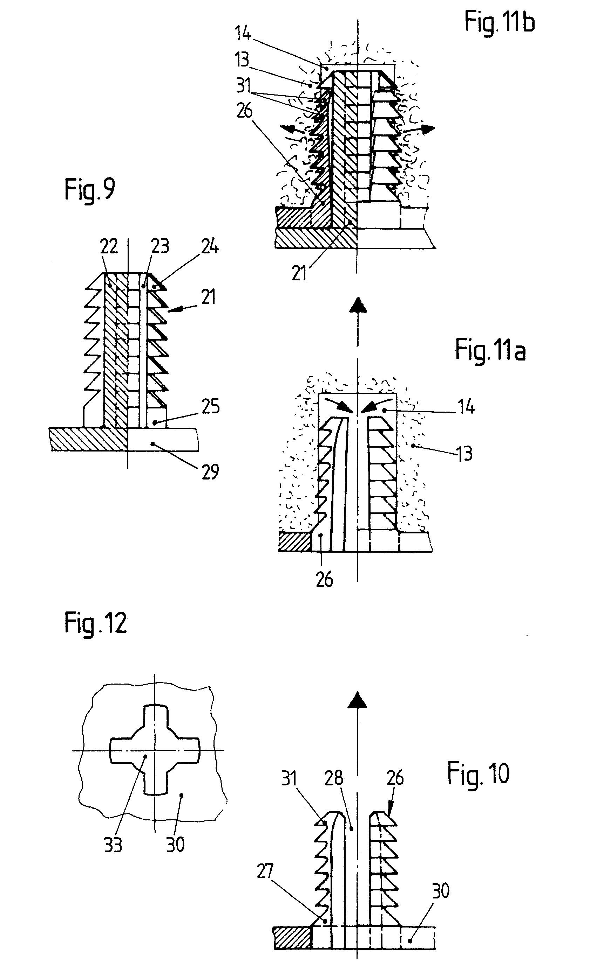

Die Konstruktion nach den Fig. 9 bis 11, die beispielsweise für einen Tibiateil einer Kniegelenkprothese bestimmt ist, unterscheidet sich von dem Ausführungsbeispiel nach den Fig. 1 bis 8 vor allem dadurch, dass der Spreizkörper 21 und der Hohlkörper 26 von der gleichen Seite her ineinandergeschoben werden.

Der Spreizkörper 21 trägt daher an seinem Kopf ein plattenförmiges Implantat 29, beispielsweise die Gleitfläche eines Kniegelenk-Tibiateils, auf der die kondylenartigen Gegenflächen des Femurteils gleitend abrollen. Der Kern 22 des Spreizkörpers 21 ist im Gegensatz zum Kern 2 des Spreizkörpers 1 am Kopf nicht erweitert, sondern hat über die ganze Länge einen konstanten Durchmesser. Während die Längsausnehmungen 23 bis zum Implantat 29 reichen, endet die Verzahnung der Vorsprünge 24 bereits vor dem Kopf oder Implantat 29 in einem Hals 25, der im implantierten Zustand von einer Auflageplatte 30 (Fig. 10) eines Hohlkörpers 26 umschlossen ist (Fig. 11b).

An der Auflageplatte 30, die dem Implantat 9 des Hohlkörpers 6 entspricht, jedoch eine – in der Form und Grösse dem Spreizkörper 21 entsprechende – Durchtrittsöffnung 33 (Fig. 12) hat, sitzen auf der einen Seite die Mantelstege 27, zwischen denen wiederum Längsschlitze 28 vorhanden sind. Die Mantelstege 27 tragen ebenfalls eine Aussenverzahnung 31 und eine nicht gezeigte Mikroverzahnung innen. Gegen das freie Ende verjüngt sich die lichte Weite der Mantelstege 27.

Die Bohrung 14 im Knochen 13 entspricht in diesem Fall dem Durchmesser der Mantelstege 27 des bei dieser Konstruktion zuerst implantierten Hohlkörpers 26.

Bei der Implantation werden die freien Enden der Mantelstege 27 elastisch zusammengepresst, wie durch Pfeile in Fig. 11a angedeutet ist, um ihre Einführung in die relativ enge Bohrung 14 zu erleichtern. Ist der Hohlkörper 26 vollständig, d.h. bis zur Auflage seiner Auflageplatte 30 auf dem Knochen 13, in die Bohrung 14 eingesetzt, so wird in der gleichen Richtung der Spreizkörper 21 durch die Bohrung 33 hindurch in den Hohlkörper 26 eingetrieben. Sobald sein Kern 22 die konische Verjüngung am freien Ende der Mantelstege 27 durchdringt, werden diese aufgespreizt und mit ihrer Verzahnung als Primär-Verankerung ähnlich wie die Verzahnung 11 des ersten Beispiels in den umgebenden Knochen 13 eingepresst.

Wie bereits erwähnt, ist es besonders zweckmässig, den Hohlkörper 6 bzw. 26 aus Metall zu fertigen, da Metalle die erforderliche Festigkeit, Elastizität und Biegeverformbarkeit haben; als Spreizkörper 1 bis 21 sind bevorzugt Kunststoffe geeignet, da sie im allgemeinen weicher als Metall sind, so dass die erwähnte Mikroverzahnung 12 leicht in ihren Kern 2 bzw. 22 eindringt und beide Teilkörper der Verankerung relativ zueinander fixiert.

**Patentansprüche**

1. Dübelartige Verankerung für eine zementfreie Fixierung von Knochenimplantaten, insbesondere von Femur- und/oder Tibiateilen von künstlichen Kniegelenken, bestehend aus einem einseitig offenen, mit Hilfe von Längsschlitzen (8) aufspreizbaren Hohlkörper (6) und einem in den Hohlkörper eintreibbaren Spreizkörper (1), wobei mindestens der Hohlkörper auf seiner Aussenmantelfläche widerhakenartige Verzahnungen trägt, dadurch gekennzeichnet, dass der Spreizkörper (1) in Form, Abmessungen und Lage an die zwischen den Längsschlitzen (8) verbliebenen Mantelstege (7) des Hohlkörpers (6) angepasste Längsausnehmungen (3) hat, zwischen denen ebenfalls mit einer widerhakenartigen Verzahnung versehene Vorsprünge (4) hervorstehen, deren Aussendurchmesser an denjenigen des Hohlkörpers (6) angepasst ist.

2. Verankerung nach Anspruch 1, dadurch gekennzeichnet, dass sich der Kern (2) des Spreizkörpers (1) gegen sein Kopfende hin stetig erweitert.

3. Verankerung nach Anspruch 1 oder 2, dadurch gekennzeichnet, dass die Innenfläche der Mantelstege (7) des Hohlkörpers (6) eine widerhakenartige Mikroverzahnung (12) trägt.

4. Verankerung nach einem der Ansprüche 1–3, dadurch gekennzeichnet, dass der Hohlkörper (6) aus Metall, insbesondere aus Titan oder einer Titanlegierung, und der Spreizkörper (1) aus Kunststoff, insbesondere Polyäthylen der Klassifikationen HDPE oder UHMW, bestehen.

## Claims

1. A plug-like anchorage for the cement-free fixing of bone implants, more particularly of femur and/or tibia parts of artificial knee joints, comprising a hollow member (6), open at one end and expandable by means of longitudinal slots (8), and an expansion member (1) which can be driven into the hollow member, at least the hollow member bearing barb-like toothing on its outer generated surface, characterised in that the expansion member (1) has longitudinal recesses (3) which are adapted in shape, dimensions and position to the generated webs (7) remaining on the hollow member (6) between the longitudinal slots (8), and between which there protrude projections (4) also provided with barb-like toothing and adapted in external diameter to the external diameter of the hollow member (6).

2. An anchorage as claimed in claim 1, characterised in that the core (2) of the expansion member (1) widens continuously towards its head end.

3. An anchorage as claimed in claim 1 or 2, characterised in that the internal surface of the generated webs (7) of the hollow member (6) bears barb-like micro-toothing (12).

4. An anchorage as claimed in any of claims 1 to 3, characterised in that the hollow member (6) is of metal, more particularly titanium or a titanium alloy, and the expansion member (1) is of synthetic plastic material, more particularly polyethylene of the HDPE of UHMW classification.

## Revendications

1. Ancrage du type cheville pour la fixation, exempte de ciment, d'implants osseux et notamment de parties fémorales et/ou tibiales d'articulations artificielles du genou, se composant d'un corps creux (6) qui est ouvert d'un côté et peut être écarté à l'aide de fentes longitudinales (8), ainsi que d'un corps d'écartement (1) pouvant être enfoncé dans le corps creux, au moins ce corps creux portant, sur sa surface externe d'enveloppement, des dentures en forme d'ardillons, caractérisé par le fait que le corps d'écartement (1) possède des évidements longitudinaux (3) qui correspondent, quant à leur forme, à leurs dimensions et à leur position, aux membrures périphériques (7) du corps creux (6) subsistant entre les fentes longitudinales (8), et entre lesquels dépassent des saillies (4) qui sont, elles aussi, dotées d'une denture en forme d'ardillons dont le diamètre externe est adapté à celui du corps creux (6).

2. Ancrage selon la revendication 1, caractérisé par le fait que le noyau (2) du corps d'écartement (1) s'évase continûment en direction de son extrémité frontale.

3. Ancrage selon la revendication 1 ou 2, caractérisé par le fait que la face interne des membrures périphériques (7) du corps creux (6) porte une microdenture (12) en forme d'ardillons.

4. Ancrage selon l'une des revendications 1–3, caractérisé par le fait que le corps creux (6) consiste en un métal, notamment en du titane ou en un alliage de titane, le corps d'écartement (1) étant constitué d'une matière plastique, notamment d'un polyéthylène de la classification HDPE ou UHMW.

Fig. 6

Fig. 5b

Fig. 7

Fig. 5a

Fig. 8

Fig. 2

Fig. 4

Fig. 1

Fig. 3

Fig. 11b

Fig. 9

Fig. 11a

Fig. 12

Fig. 10